# EUROPÄISCHE PATENTSCHRIFT

(11) **EP 1 289 476 B1**
(45) Veröffentlichungstag und Bekanntmachung des Hinweises auf die Patenterteilung: **11.05.2005**
(21) Anmeldenummer: 01947348.7
(22) Anmeldetag: 07.06.2001
(51) Int. Cl.: A61K 7/00, A61K 7/16

(54) **THIXOTROPE MUND- UND ZAHNPFLEGEMITTEL**
THIXOTROPIC ORAL AND DENTAL CARE AGENTS
AGENTS THIXOTROPES POUR L'HYGIENE BUCCALE ET DENTAIRE

(30) Priorität: 16.06.2000 DE 10028974
(43) Veröffentlichungstag der Anmeldung: 12.03.2003
(73) Patentinhaber: Henkel Kommanditgesellschaft auf Aktien, 40589 Düsseldorf (DE)
(72) Erfinder: KROPF, Christian, 40721 Hilden (DE); BRÜNINGHAUS, Ulrike, 40789 Monheim (DE)
(86) Internationale Anmeldenummer: PCT/EP2001/006425
(87) Internationale Veröffentlichungsnummer: WO 2001/095864

(56) Entgegenhaltungen:
- EP-A- 0 314 050
- EP-A- 0 733 357
- EP-A- 0 947 203
- WO-A-01/27236
- US-A- 3 963 832
- US-A- 5 302 373
- US-A- 5 330 749

## Beschreibung

Die Erfindung betrifft thixotrope Mund- und Zahnpflegemittel mit einem Gehalt an einer oder mehreren nanopartikulären anorganischen Verbindungen aus der Gruppe der Metall -silicate.

In der Mund- und Zahnpflege werden neben Zahnpasten vielfach flüssige Zubereitungen eingesetzt, bei welchen der Reinigungs- und Pflegeeffekt durch den Kontakt der Zubereitung mit der Mundhöhle erzielt wird, ohne daß es eines zusätzlichen Hilfsmittels wie einer Zahnbürste bedarf. Diese Zubereitungen, die üblicherweise als Mundwässer bezeichnet werden, bieten dadurch einen besonderen Anwendungskomfort und gewährleisten darüber hinaus, daß auch schwer zugängliche Bereiche der Mundhöhle, insbesondere an den Zähnen, mit der Zubereitung in Kontakt kommen.

Während Mundwässer in der Vergangenheit lediglich zur Erfrischung des Mund- und Rachenraumes und zur Verbesserung des Mundgeruchs dienten, übernehmen sie heute Aufgaben wie z. B. eine plaqueanlösende Wirkung, und sie dienen als Träger von Antikarieswirkstoffen oder antibakteriellen Wirkstoffen zur Plaquebekämpfung. Mundwässer werden als dünnflüssige wäßrige oder wäßrig-alkoholische Formulierungen angeboten bzw. als wasserverdünnbare Konzentrate. Durch die Applikation in Form einer dünnflüssigen Zubereitung wird zwar einerseits eine gute Verteilbarkeit und ein guter Kontakt mit der Mundhöhle erreicht, nachteilig ist in bestimmten Fällen jedoch die kurze Kontaktzeit zwischen dem Mundwasser und insbesondere den Zähnen. Ein intensiver Kontakt findet nur während des Spülvorgangs im Mund statt, und sobald dieser eingestellt wird, verbleibt lediglich einer dünner Film des Mundwassers auf den Zähnen. Dieser Kontakt ist für bestimmte in der Mund- und Zahnpflege eingesetzte Wirkstoffe zu kurz und zu wenig intensiv, so daß solche Wirkstoffe in Mundwässem nicht einsetzbar sind bzw. keine oder nur unbefriedigende Wirkung entfalten können. Ein Beispiel dafür sind remineralisierende Wirkstoffe, welche zunächst auf die Zahnoberfläche aufziehen müssen und anschließend auf dem Weg einer Biomineratisation zur Reparatur und zum Aufbau von Zahnschmelz beitragen.

Ein weiterer Nachteil der herkömmlichen Mundwässer besteht darin, daß es aufgrund ihrer Dünnflüssigkeit sehr schwierig ist, schwerlösliche Stoffe stabil in die Formulierungen einzuarbeiten, und es kommt in diesem Fall häufig zu Sedimentationserscheinungen und somit zu unbefriedigenden Lagerstabilitäten derartiger Produkte.

In der US 5,455,023 sind Mundwässer beschrieben, in die wegen seiner positiven Eigenschaften z. B. als Deodorans und Puffersubstanz eine besonders hohe Konzentration an Natriumbicarbonat eingearbeitet werden sollte, welches in herkömmlichen Mundwässern nur in geringer Konzentration stabil formulierbar ist. Erfindungsgemäß wurde diese Aufgabe dadurch gelöst, daß das Natriumbicarbonat in Form von Partikeln mit einem mittleren Teilchendurchmesser von 500 bis 5000 nm eingearbeitet wurde. Zur zusätzlichen Stabilisierung der Formulierungen ist die optionale Zugabe von polymeren Verdickungsmitteln beschrieben, als deren weiterer Effekt eine verlängerte Einwirkdauer der Mundwasser-Rückstände beschrieben wird. Mit den Verdickungsmitteln können die Zubereitungen auf eine hohe Viskosität eingestellt werden und weisen dann thixotrope Eigenschaften auf.

US-A-5,302,373 und US-A-5,330,749 offenbaren Mundwaschzusammensetzungen, die in Form konzentrierter wässriger ethanolischer Formulierungen vorliegen. Die Mundwaschzusammensetzungen enthalten feinpartikuläre Alkalimetallbicarbonat und kolloidales Zinkoxid, die zur Desodorierung und Pufferung der Mundwaschformulierung eingesetzt werden.

EP-A2-947203 offenbart eine Kontaktlinsenzusammensetzung, bestehend aus einem wässrigen Medium und synthetischen Smektitpartikeln mit einer Partikelgröße von maximal 200 nm.

Die JP-A 09241152 beschreibt Chitin- oder Chitosanhaltige thixotrope Öl-in Wasser-Emulsionen, welche für entzündungshemmende Zusammensetzungen im Mundbereich angewendet werden. Aufgrund der thixotropen Eigenschaften werde eine höhere Verweilzeit am Wirkort erreicht. Dem Fachmann ist jedoch bekannt, daß die Formulierung von Chitin- und Chitosanhaltigen Rezepturen aufgrund der problematischen Löslichkeit dieser Biopolymere mit erheblichen Schwierigkeiten verbunden und nur in eng begrenzten Bereichen möglich ist, insbesondere im Falle alkoholhaltiger Formulierungen.

Die aus der Kosmetik bekannten Verdickungs- und Thixotropiermittel weisen Eigenschaften auf, die für den Einsatz in Mundwässem nicht vorteilhaft sind. So benötigt man teilweise beträchtliche Mengen dieser Stoffe, um die gewünschte Konsistenz der thixotropen Formulierung zu erzielen, und teilweise ist eine zu hohe, für den Verbraucher bei der Anwendung nicht akzeptale mechanische Energie, beispielsweise durch Schütteln, zur Verflüssigung der Formulierung erforderlich. Ein besonderes Problem liegt jedoch darin, daß die Zeitdauer für die Verflüssigung und andererseits die Wiederverfestigung der thixotropen Formulierung zu lange ist. Insbesondere zur Anwendung thixotroper Formulierungen in sprühbaren Produkten sind aus dem Stand der Technik keine befriedigenden Lösungen bekannt.

Aufgabe der vorliegenden Erfindung war es, ein thixotropes Mund- und Zahnpflegemittel bereitzustellen, das im Ruhezustand als Gel mit Fließgrenze vorliegt, sich unter verbraucherrelevanten Anwendungsbedingungen, beispielsweise durch Schütteln vor Gebrauch oder durch Versprühen, reversibel verflüssigen läßt und das bereits nach kurzer Zeit, beispielsweise innerhalb weniger Sekunden oder sogar unmittelbar nach Beendigung der Scherkrafteinwirkung, wieder ein Gel bildet.

Überraschenderweise wurde gefunden, daß bestimmte anorganische Stoffe in nanopartikulärer Form besonders gut zur Thixotropierung von Mund- und Zahnpflegeprodukten wie beispielsweise von Mundwässem geeignet sind.

Gegenstand der Erfindung ist ein thixotropes Mund- und Zahnpflegemittel, das eine oder mehrere nanopartikuläre anorganische Verbindungen aus der Gruppe der Metall silicate umfaßt sowie zusätzlich mindestens einen mineralisierenden Wirkstoff.

Im Sinne der Erfindung als Metalle bevorzugt sind Alkalimetalle, Calcium, Magnesium. Aluminium, Titan, Zirkon und Zink, wobei Magnesium besonders bevorzugt ist.

Weiterhin bevorzugt ist, daß die in den erfindungsgemäßen Mitteln enthaltenen nanopartikulären anorganischen Verbindungen wenig wasserlöslich sind. Als wenig wasserlöslich sollen solche Verbindungen verstanden werden, die in Wasser bei 20°C zu weniger als 1 g/l und vorzugsweise zu weniger als 1 mg/l löslich sind.

Die mittlere Teilchengröße der nanopartikulären Verbindungen beträgt üblicherweise 1 bis 200 nm, vorzugsweise 5 bis 100 nm, und besonders bevorzugt 10 bis 50 nm, wobei der Wert sich auf den Teilchendurchmesser in der Längsrichtung, d.h. in der Richtung der größten Ausdehnung der Teilchen bezieht.

Der Gehalt an einer oder mehreren nanopartikulären anorganischen Verbindungen aus der Gruppe der Metall silicate beträgt üblicherweise von 0,1 bis 20 Gew.-%, vorzugsweise 0,2 bis 10 Gew.-%, und besonders bevorzugt 0,5 bis 5 Gew.-%, wobei die %-Angaben jeweils als Summe des Gewichts der nanopartikulären anorganischen Verbindungen bezogen auf das Gesamtgewicht der Mittel zu verstehen sind.

Geignete nanopartikuläre Silicate sind z. B. Alumosilicate wie Zeolithe sowie Magnesiumsilicate. Bevorzugte Silicate sind die Schichtsilicate (Phyllosilicate), insbesondere Bentonite (enthalten als Hauptminerale Smektite, v.a. Montmorillonit), Montmorillonite (Al₂[(OH)₂/Si₄O₁₀] · n H₂O bzw. Al₂O₃ · 4 SiO₂ · H₂O · n H₂O, zu den dioktaedrischen (Glimmer) Smektiten gehörendes Tonmineral), Kaolinit (Al₂[(OH)₄/Si₂O₅] bzw. Al₂O₃ · 2 SiO₂ · 2 H₂O, triklines Zweischicht-Tonmineral (1:1-Phyllo-Silicat)), Talk (hydratisiertes Magnesiumsilicat der Zusammensetzung Mg₃[(OH)₂/Si₄O₁₀] oder 3 MgO · 4 SiO₂ · H₂O) und besonders bevorzugt Hectorite (z.B. M⁺_{0,3}(Mg_{2,7}Li_{0,3})[Si₄O₁₀(OH)₂], M⁺ meist = Na⁺, zu den Smektiten gehörendes, dem Montrnorillonit ähnliches, monoklines Tonmineral).

In einer besonderen Ausführungsform der Erfindung werden nanopartikuläre anorganische Verbindungen mit einer spezifischen Oberfläche von mehr als 200 m²/g eingesetzt. Eine bevorzugte derartige nanopartikuläre Verbindung ist Magnesiumsilikat vom Schichtsilikat-Typ mit einer spezifischen Oberfläche von 200 bis 500 m²/g, insbesondere 300 bis 400 m²/g. Dieses Material ist preiswert in großen Mengen verfügbar. Das Produkt ist unter den Handelsnamen Optigel® SH (Süd-Chemie AG) sowie Laponite® XLG (Laporte Ltd.) verfügbar.

In einer weiteren besonderen Ausführungsform der Erfindung sind die nanopartikulären anorganischen Verbindungen mit einem oder mehreren Oberflächenmodifikationsmitteln modifiziert. Unter Modifizierung ist die Belegung der Partikeloberfläche mit organischen Verbindungen zu verstehen, die über chemische Bindungen oder physikalische Kräfte mit der Oberfläche der Partikel wechselwirken.

Als Oberflächenmodifikationsmittel für die Nanopartikel eignen sich alle ein- und mehrbasischen Carbonsäuren und Hydroxycarbonsäuren mit 2 bis 18 C-Atomen, also z.B. Essigsäure, Propionsäure, Oxalsäure, Glutarsäure, Maleinsäure, Bernsteinsäure, Phthalsäure, Adipinsäure, Korksäure, Palmitinsäure und Stearinsäure. Bevorzugt geeignet sind die Hydroxycarbonsäuren und Fruchtsäuren wie z.B. Glycolsäure, Milchsäure, Zitronensäure, Äpfelsäure, Weinsäure und Gluconsäure. Besonders bevorzugt wird als Carbonsäure eine Hydroxycarbonsäure aus der Gruppe Milchsäure, Zitronensäure, Äpfelsäure und Weinsäure eingesetzt.

Die Oberflächenmodifikation der anorganischen Nanopartikel erfolgt bevorzugt durch Behandlung mit einer wäßrigen Lösung einer Carbon- oder Hydroxycarbonsäure in der Weise, daß die Nanopartikel mit einer Lösung von 0,05 bis 0,5 mol der Carbonsäure pro Mol der nanopartikulären anorganischen Verbindung behandelt werden. Diese Behandlung erfolgt bevorzugt über einen Zeitraum von 1 bis 24 Stunden bei einer Temperatur von wenigstens 20 °C, bevorzugt aber bei der Siedetemperatur des Wassers bei Normaldruck (100 °C). Bei Anwendung von Druck kann die Behandlung auch bei Temperaturen oberhalb 100 °C in entsprechend kürzerer Zeit erfolgen.

Durch die Behandlung mit den Carbonsäuren oder Hydroxycarbonsäuren wird die Oberfläche der Nanopartikel modifiziert. Es wird angenommen, daß die Carbonsäuren oder Hydroxycarbonsäuren esterartig an die Oberfläche der Nanopartikel gebunden werden.

Die oberflächenmodifizierten Nanopartikel werden aus dem Reaktionsgemisch bevorzugt durch Entwässerung isoliert. Zu diesem Zweck wird die Dispersion vorzugsweise einer Gefriertrocknung unterworfen. Dabei wird das Lösungsmittel bei tiefer Temperatur im Hochvakuum absublimiert.

Nach diesem Verfahren modifizierte anorganische Nanopartikel enthalten zwischen 1 und 30 Gew.-%, bevorzugt zwischen 5 und 20 Gew.-%, des organischen Oberflächenmodifikationsmittels bezogen auf das Gesamtgewicht der oberflächenmodifizierten anorganischen Nanopartikel.

Zur Oberflächenmodifikation der Nanopartikel können auch funktionelle Silane des Typs (OR)₄₋ₙSiRₙ (R = org. Reste mit funktionellen Gruppen wie Hydroxy, Carboxy, Ester, Amin, Epoxy, etc.), quartäre Ammoniumverbindungen, Phosphonsäuren oder Aminosäuren eingesetzt werden. Je nach Polarität der Modifikationsmittel wird die oben beschriebene Modifikation in Wasser oder in organischen Lösungsmitteln (Alkohole, Ether, Ketone, Kohlenwasserstoffe, etc.) durchgeführt, wobei die Reaktionsbedingungen analog zu denen in Wasser zu wählen sind. Schichtsilikate wie z.B. Hectorite können auch einem Ionenaustausch unterzogen werden, wobei Kationen wie z.B. quartäre Ammoniumverbindungen zwischen die Schichten des Materials eingebaut werden. Als weitere Oberflächenmodifikationsmittel sind beispielsweise Gelatine, Stärke, Dextrin, Dextran, Pektin, Gummi arabicum, Kasein, Gummen, Polyvinylalkohole, Polyethylenglykole, Polyvinylpyrrolidon, Polyvinylbutyrale, Methylcellulose, Carboxymethylcellulose, Hydroxypropylcellulose geeignet oder auch Tenside und Emulgatoren wie z.B. Fettalkoholpolyglykolether, Fettalkoholpolyglycoside, Fettsäurealkanolamide, Glycerolester, Sorbitanester oder alkoxylierte Ester und deren Derivate.

Die erfindungsgemäßen Mittel können weiterhin Inhaltsstoffe enthalten, wie sie für Mund- und Zahnpflegemittel üblich sind, wie beispielsweise Tenside, Geschmacksstoffe, Lösungsvermittler, mundhygienische Wirkstoffe, Farbstoffe und Trübungsmittel.

Als Tenside können anionische, kationische, zwitterionische, ampholytische und nichtionogene oberflächenaktive Substanzen mit einer Wasserlöslichkeit von wenigstens 1 Gew.-% (20°C) in Mengen von 0,1 bis 5 Gew.-%, bevorzugt von 0,1 bis 1 Gew.-%, eingesetzt werden.

Als oberflächenaktive Stoffe sind dabei bevorzugt solche Stoffe zu verstehen, die eine lipophile, lineare Alkyl- oder Acylgruppe mit 10 bis 22 C-Atomen und eine wasserlöslich-machende ionische Gruppe, z.B. eine Sulfat-, Sulfonat-, Phosphat-, Carboxylat- oder z.B. eine Trimethylammoniumgruppe oder eine Acetobetain-Gruppe oder eine nichtionische Polyhydroxyalkyl- oder Polyoxyethylengruppe enthalten. Beispiele für geeignete ionische Tenside sind z.B. Natriumlaurylsulfat, Natrium-lauroylisethionat, Cetyltrimethylammoniumchlorid, Lauryl-trimethylammonium-acetobetain, Lauroylamidopropyldimethyl-ammonium-acetobetain. Bevorzugt sind aber nichtionische oberflächenaktive Stoffe enthalten, als Beispiele werden Anlagerungsprodukte von Ethylenoxid an Fettalkohole, an Fettsäuren, an Fettsäuremonoglyceride, an Sorbitanfettsäureester, an Propylenglycol-monofettsäureester oder an Methylglycosid-monofettsäureester genannt. Weitere, bevorzugt geeignete nichtionische Tenside sind Alkyl-(oligo)-glycoside.

Bezüglich des Glycosidrestes gilt, daß sowohl Monoglycoside (x = 1), bei denen ein Hexoserest glycosidisch an einen Fettalkohol mit 8 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl-(oligo)-glycosid ein Alkyl-(oligo)-glucosid der Formel RO(C₆H₁₀O)ₓ-H, in der R eine Alkylgruppe mit 12 bis 14 C-Atomen ist und x einen Mittelwert von 1 bis 4 hat.

Außer den genannten Alkylglucosid-Tensiden können auch andere nichtionische, ampholytische und kationische Tenside enthalten sein. Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäure-sorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinotsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

Die erfindungsgemäßen Mittel enthalten als Lösungsvermittler für gegebenenfalls enthaltene Aromaöle bevorzugt Anlagerungsprodukte von 20 bis 60 Mol Ethylenoxid an gehärtetes oder ungehärtetes Ricinusöl (d. h. an Oxystearinsäure- oder Ricinolsäure-triglycerid), an Glycerin-mono- und/oder - distearat oder an Sorbitanmono- und/oder -distearat.

Als Geschmacksstoffe können z. B. Süßungsmittel und/oder Aromaöle enthalten sein. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krausenminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton.

Als Süßungsmittel eignen sich z.B. Saccharin-Natrium, Natrium-cyclamat, Acesulfam-K, Aspartam, Lactose, Maltose, Fructose, Glycerin, Sorbit, Mannit oder Xylit.

Schließlich können die erfindungsgemäßen Mittel mundhygienische und therapeutische Wirkstoffe enthalten wie z. B.
- karieshemmende Fluorverbindungen, z. B. Natriumfluorid, Zinnfluorid, Natriummonofluorophosphat oder Aminfluorid
- Antizahnsteinwirkstoffe, z. B. Organophosphonate, Natriumpyrophosphat, Natriumtripolyphosphat
- wundheilende und entzündungshemmende Stoffe wie z.B. Allantoin, Harnstoff, Azulen bzw. Kamillenextrakt,
- antibakterielle, plaquehemmende Stoffe, wie z.B. Chlorhexidin oder Triclosan.

Die erfindungsgemäßen Mittel enthalten einen oder mehrere remineralisierende Wirkstoffe. Als remineralisierende Wirkstoffe kommen beispielsweise Fluoride wie Cetytaminhydrofluorid sowie Calciumsalze in Betracht.

Besonders bevorzugt sind die remineratisierenden Wirkstoffe ausgewählt aus der Gruppe, die gebildet wird von wenig wasserlöslichen Phosphaten, Fluoriden und Fluorophosphaten des Calciums, insbesondere solchen mit einem mittleren Teilchendurchmesser im Bereich von 5 bis 300 nm, welche zusätzlich von Tensiden oder Schutzkolloiden ummantelt oder als Komposite mit Proteinkomponenten vorliegen können. Solche im Sinne der vorliegenden Erfindung besonders geeigneten Calciumsalze sind beispielsweise die in der Patentanmeldung DE 19858662.0 beschriebenen nanopartikulären Calciumsalze sowie die in der Patentanmeldung DE 19930335.5 beschriebenen Kompositmaterialien aus Calciumsalzen und Proteinkomponenten. Im Sinne der vorliegenden Erfindung insbesondere bevorzugt ist es, wenn als remineralisierender Wirkstoff in den erfindungsgemäßen Mitteln nanopartikulärer Apatit, Hydroxylapatit oder Fluorapatit enthalten ist.

Die remineralisierenden Wirkstoffe werden bevorzugt in Form von Nanopartikeln eingesetzt, da hier wegen der großen spezifischen Oberfläche der Nanopartikel eine besonders gute Haftung zum natürlichen Zahnmaterial besteht und die feinen Partikel auch nach dem Ausspülen auf dem Zahnschmelz haften bleiben. Sie dringen bevorzugt in kleine Unebenheiten wie Risse oder Fissuren ein und bewirken dort eine schnelle Remineralisierung.

Der Gehalt der remineratisierenden Wirkstoffe in den erfindungsgemäßen Mitteln beträgt üblicherweise von 0,1 bis 10 Gew.-%, und vorzugsweise 0,5 bis 5 Gew.-%, bezogen auf das Gesamtgewicht der Mittel.

Die erfindungsgemäßen Zubereitungen können weiterhin zwischen 0,5 und 15 Gew.-% Ethanol enthalten.

Ein weiterer Gegenstand der Erfindung ist die Verwendung eines thixotropen Mund- und Zahnpflegemittels gemäß Anspruch 9.

Die erfindungsgemäßen Mittel eignen sich insbesondere als Mundspülungen oder Mundwässer. Gegenstand der Erfindung ist daher auch die Verwendung der erfindungsgemäßen Mittel als Mundspülung oder Mundwasser.

Ein weiterer Gegenstand ist die Verwendung eines thixotropen Mund- und Zahnpflegemittels gemäß Anspruch 10.

Die erfindungsgemäßen Mittel lassen sich - sowohl mit einer Sprühpumpe wie auch als Aerosol - versprühen.

Im Ruhezustand bilden die erfindungsgemäßen Mittel ein festes Gel, erst durch die Bewegungen im Mund tritt Verflüssigung ein zu einem wäßrig dünnen System. Ohne Spül-Bewegungen tritt erneut sofortige Verfestigung ein. Dadurch verbleiben die Wirkbestandteile länger auf den Zähnen und können adhäsiv haften oder aufgenommen werden. Dies ist von besonderer Bedeutung beispielsweise für Wirkstoffe zur Remineralisierung der Zähne und für antibakterielle Wirkstoffe.

Die in den erfindungsgemäßen Mitteln eingesetzten nanopartikutären anorganischen Verbindungen bewirken eine sehr effiziente Thixotropierung, d. h. bereits mit relativ geringen Einsatzmengen und unter geringen Kosten lassen sich Zusammensetzungen mit einer sehr ausgeprägten Thixotropie herstellen.

Die erfindungsgemäßen thixotropen Mittel ermöglichen es, auch schwerlösliche und partikuläre Stoffe stabil in die Formulierungen einzuarbeiten, und es kommt weder zu Sedimentationserscheinungen noch zu unbefriedigenden Lagerstabilitäten derartiger Mittel.

Besonders vorteilhaft ist es, daß einerseits die Verflüssigung der thixotropen Mittel nach Einbringung von mechanischer Scherenergie und andererseits die Wiederverfestigung im Ruhezustand sehr sehnell erfolgt, beispielsweise innerhalb weniger Sekunden oder sogar unmittelbar nach Beendigung der Scherkrafteinwirkung. Dies ist ganz besonders wertvoll zur Anwendung derartiger thixotroper Mittel in sprühbaren Produkten. So sind die erfindungsgemäßen Mittel beispielsweise mit Hilfe von Pumpzerstäubem applizlerbar, wobei die Verflüssigung alleine durch die Betätigung des Pumpzerstäubers geschehen kann, und ohne daß der Zerstäuber vorher geschüttelt wurde. Sobald sich der Sprühnebel des Mittels auf der Zahnoberfläche niedergeschlagen hat, verfestigt er sich.

Ein weiterer Vorteil der in den erfindungsgemäßen Mitteln zur Thixotropierung eingesetzten nanopartikulären anorganischen Verbindungen besteht darin, daß sie sich in der Rezeptur weitgehend neutral verhalten, also die übrigen in der Rezeptur vorhandenen Inhaltsstoffe nicht negativ beeinflussen.

Ein zusätzlicher Vorteil der erfindungsgemäßen Mittel besteht in ihrer Transparenz, die durch die nanopartikulären anorganischen Verbindungen nicht beeinträchtigt wird.

Zur Herstellung der erfindungsgemäßen Mittel werden die Inhaltsstoffe vermengt, ggf. unter Anwendung von Ultraschall.

Die folgenden Beispiele sollen die vorliegende Erfindung verdeutlichen, ohne sie einzuschränken. Alle Prozentanteile sind, soweit nicht anders angegeben, auf das Gesamtgewicht der Zubereitungen bezogen.

### Beispiele:

### Beispiel 1: Mundgel

| Inhaltsstoff | Gehalt in Gew.-% |
|---|---|
| Ethanol | 2,4 |
| Sorbit 70% | 4 |
| Cremophor RH 60 | 2 |
| Chlorhexidin | 0,2 |
| Plantacare 1200 | 1 |
| Aroma | 0,2 |
| Farbstoff | 0,001 |
| Hectorit (Laponite XLG) | 4 |
| nanopartikulärer Hydroxylapatit | 2 |
| Wasser | ad 100 |
| Cremophor RH 60: hydriertes Rizinusöl mit 60 EO | |
| Plantacare 1200: C12-C16-Alkylglykosid | |
| nanopartikulärer Hydroxylapatit: hergestellt nach Beispiel 1.1. der Patentanmeldung DE 19858662.0, wobei Plantacare 1200 durch Cremophor RH 60 ersetzt wurde; der in obiger Tabelle angegebene Gehalt bezieht sich auf den wasserfreien Hydroxylapatit, das aufgrund der Herstellung mitgeführte Cremophor RH 60 ist in der Tabelle separat berücksichtigt. | |

### Beispiel 2: Versprühbares Mundgel

| Inhaltsstoff | Gehalt in Gew.-% |
|---|---|
| Ethanol | 5 |
| Sorbit 70% | 3 |
| Chlorhexidin | 0,2 |
| APG 220 UPW | 0,5 |
| Cremophor RH 60 | 0,1 |
| Aroma | 0,001 |
| Farbstoff | 0,001 |
| Hectorit (Laponite XLG) | 2 |
| nanopartikulärer Hydroxylapatit | 1 |
| Zitronensäure | 0,05 |
| Wasser | ad 100 |
| APG 220 UPW: C8-10 Alkyl-1,5-glucosid, AS-Gehalt 62-65% | |

Das Gel hat einen pH-Wert von 7,3 und weist im Ruhezustand eine schnittfeste Konsistenz auf.

## Patentansprüche

1. Thixotropes Mund- und Zahnpflegemittel, umfassend eine oder mehrere nanopartikuläre anorganische Verbindungen aus der Gruppe der Metallsilicate, **dadurch gekennzeichnet, dass** es zusätzlich mindestens einen remineralisierenden Wirkstoff enthält.

2. Mittel nach Anspruch 1, **dadurch gekennzeichnet, dass** das Metall ausgewählt ist aus der Gruppe gebildet von Alkalimetallen, Calcium, Magnesium, Aluminium, Titan, Zirkon und Zink.

3. Mittel nach einem der Ansprüche 1 oder 2, **dadurch gekennzeichnet, dass** die mittlere Teilchengröße der nanopartikulären Verbindungen 1 bis 200 nm, vorzugsweise 5 - 100 nm, besonders bevorzugt 10 bis 50 nm, beträgt.

4. Mittel nach einem der Ansprüche 1 bis 3, **dadurch gekennzeichnet, dass** es eine oder mehrere nanopartikuläre Verbindungen in einer Menge von insgesamt 0,1 bis 20 Gew.-%, vorzugsweise 0,2 bis 10 Gew.-%, besonders bevorzugt 0,5 bis 5 Gew.-% enthält.

5. Mittel nach einem der Ansprüche 1 bis 4, **dadurch gekennzeichnet, dass** es Hectorit enthält.

6. Mittel nach einem der Ansprüche 1 bis 5, **dadurch gekennzeichnet, dass** die nanopartikuläre anorganische Verbindung mit mindestens einem Oberflächenmodifikationsmittel modifiziert ist.

7. Mittel nach einem der Ansprüche 1 bis 6, **dadurch gekennzeichnet, dass** der remineralisierende Wirkstoff ausgewählt ist aus der Gruppe, die gebildet wird von wenig wasserlöslichen Phosphaten, Fluoriden und Fluorophosphaten des Calciums, insbesondere mit einem mittleren Teilchendurchmesser im Bereich von 5 bis 300 nm.

8. Mittel nach einem der Ansprüche 1 bis 7, **dadurch gekennzeichnet, dass** der remineralisierende Wirkstoff in einer Menge von 0,1 bis 10 Gew.-%, vorzugsweise 0,5 bis 5 Gew.-% enthalten ist, bezogen auf das Gesamtgewicht des Mittels.

9. Verwendung eines thixotropen Mund- und Zahnpflegemittels umfassend eine oder mehrere nanopartikuläre anorganische Verbindungen aus der Gruppe der Metallsilicate als Mundwasser.

10. Verwendung eines thixotropen Mund- und Zahnpflegemittels umfassend eine oder mehrere nanopartikuläre anorganische Verbindungen aus der Gruppe der Metallsilikate als sprühbares Mund- und Zahnpflegemittel, insbesondere als sprühbares Mundwasser.

## Claims

1. A thixotropic oral hygiene and dental care preparation containing one or more nanoparticulate inorganic compounds from the group of metal silicates, **characterized in that** it additionally contains at least one remineralizing component.

2. A preparation as claimed in claim 1, **characterized in that** the metal is selected from the group consisting of alkali metals, calcium, magnesium, aluminium, titanium, zirconium and zinc.

3. A preparation as claimed in claim 1 or 2, **characterized in that** the mean particle size of the nanoparticulate compounds is in the range from 1 to 200 nm, preferably in the range from 5 to 100 nm and more particularly in the range from 10 to 50 nm.

4. A preparation as claimed in any of claims 1 to 3, **characterized in that** it contains one or more nanoparticulate compounds in a total quantity of 0.1 to 20% by weight, preferably 0.2 to 10% by weight and more particularly 0.5 to 5% by weight.

5. A preparation as claimed in any of claims 1 to 4, **characterized in that** it contains hectorite.

6. A preparation as claimed in any of claims 1 to 5, **characterized in that** the nanoparticulate inorganic compound is modified with at least one surface modifier.

7. A preparation as claimed in any of claims 1 to 6, **characterized in that** the remineralizing component is selected from the group consisting of poorly water-soluble phosphates, fluorides and fluorophosphates of calcium, more particularly with a mean particle diameter of 5 to 300 nm.

8. A preparation as claimed in any of claims 1 to 7, **characterized in that** the remineralizing component is present in a quantity of 0.1 to 10% by weight and preferably in a quantity of 0.5 to 5% by weight, based on the total weight of the preparation.

9. The use of a thixotropic oral hygiene and dental care preparation containing one or more nanoparticulate inorganic compounds from the group of metal silicates as a mouth wash.

10. The use of a thixotropic oral hygiene and dental care preparation containing one or more nanoparticulate inorganic compounds from the group of metal silicates as a sprayable oral hygiene and dental care preparation, more particularly as a sprayable mouth wash.

## Revendications

1. Agent de soins bucco-dentaires, thixotrope, comprenant un ou plusieurs composés inorganiques nanoparticulaires, issus du groupe des silicates métalliques, **caractérisé en ce qu'**il contient en outre au moins une substance active re-minéralisante.

2. Agent selon la revendication 1, **caractérisé en ce que** le métal est choisi dans le groupe formé par les métaux alcalins, le calcium, le magnésium, l'aluminium, le titane, le zirconium et le zinc.

3. Agent selon l'une quelconque des revendications 1 ou 2, **caractérisé en ce que** la taille moyenne de particule des composés nanoparticulaires est de 1 à 200 nm, de préférence de 5 à 100 nm, de manière particulièrement préférée de 10 à 50 nm.

4. Agent selon l'une quelconque des revendications 1 à 3, **caractérisé en ce qu'**il contient un ou plusieurs composés nanoparticulaires en une quantité au total de 0,1 à 20% en poids, de préférence de 0,2 à 10% en poids, de manière particulièrement préférée de 0,5 à 5% en poids.

5. Procédé selon l'une quelconque des revendications 1 à 4, **caractérisé en ce qu'**il contient une hectorite.

6. Agent selon l'une quelconque des revendications 1 à 5, **caractérisé en ce que** le composé inorganique nanoparticulaire est modifié par au moins un agent de modification de surface.

7. Agent selon l'une quelconque des revendications 1 à 6, **caractérisé en ce que** la substance active re-minéralisante est choisie dans le groupe qui est formé par des phosphates, fluorures et fluorophosphates du calcium, peu hydrosolubles, en particulier ayant un diamètre moyen de particule dans la plage de 5 à 300 nm.

8. Agent selon l'une quelconque des revendications 1 à 7, **caractérisé en ce que** la substance active re-minéralisante est contenue en une quantité de 0,1 à 10% en poids, de préférence de 0,5 à 5% en poids, par rapport au poids total de l'agent.

9. Utilisation d'un agent de soins bucco-dentaires, thixotrope, comprenant un ou plusieurs composés inorganiques nanoparticulaires issus du groupe des silicates métalliques, sous forme de bain de bouche.

10. Utilisation d'un agent de soins bucco-dentaires, thixotrope, comprenant un ou plusieurs composés inorganiques nanoparticulaires issus du groupe des silicates métalliques, sous la forme d'agent de soins bucco-dentaires, pulvérisable, en particulier sous la forme de bain de bouche, pulvérisable.
